Europäisches Patentamt

European Patent Office

Office européen des brevets

(19)

(11) Publication number: 0 141 536

A1

(12) **EUROPEAN PATENT APPLICATION**

(21) Application number: **84306772.9**

(22) Date of filing: **04.10.84**

(51) Int. Cl.⁴: **A 61 B 17/32**

(30) Priority: **13.10.83 IL 69962**

(43) Date of publication of application:
**15.05.85 Bulletin 85/20**

(84) Designated Contracting States:
**DE FR GB**

(71) Applicant: **WERTHEIMER LTD.**
**Industrial Zone**
**Naharya 22 102(IL)**

(72) Inventor: **Resnick, Semion**
**46b Deganiot Street**
**Kiriat Tivon 36 000(IL)**

(72) Inventor: **Goldman, Jacob**
**55 Jabotinsky Street**
**Naharya 22 102(IL)**

(74) Representative: **Miller, Joseph et al,**
**J. MILLER & CO. Lincoln House 296-302 High Holborn**
**London WC1V 7JH(GB)**

(54) **An abdominal wall aperturing device.**

(57) An abdominal wall aperturing device comprises an elongated body having a roatable handle at one end thereof. A shaft axially extends through said body and has an end extendable beyond the body to enable an anvil to be attached thereto. The other end of the shaft is threadably coupled to the handle so that when the latter rotates an axial motion is imparted to the shaft. A knife-surrounding cartridge is attachable to the body end through which the shaft extends to the anvil. Means are included within and outside the body to impart a rotational and/or axial motion to the knife to advance it through a compressed abdominal wall to cut an aperture therein.

EP 0 141 536 A1

Fig.6

An abdominal wall aperturing device

BACKGROUND OF THE INVENTION

1. Field of the Invention

This invention relates to an abdominal wall aperturing device, particularly for use in making an aperture in the abdominal wall, such as in colostomies and ileostomies.

2. Description of the Prior Art

In general, such apertures (of a generally circular shape) have been made using an operating scalpel and scissors. But such methods have not been conducive to obtaining a clean aperture of accurately predetermined size and shape. It has also been found to be difficult, if not impossible, to avoid relative displacement of the various abdominal wall layers (skin, facia, muscle and peritoneum).

There has been proposed to use, for this purpose, an abdominal wall punch having an L-shaped body with a lower arm which is inserted through an incision and a circular cutter supported from another arm which can be pushed and turned through the tissue layers above the lower arm. This proposed punch is of cumbrous construction, not lending itself to easy use by the operating surgeon. Furthermore, with such a punch the muscle layers of the abdominal wall are cut through, thereby severing the muscles in the region of the aperture and this has been found to be undesirable.

- 2 -

Applicants have previously proposed a device comprising a body member, with an elongated shaft to which an anvil is attachable. A cutting knife having a substantially circular cutting edge, which is releasably held with respect to the body member, is axially or linearly displacable toward the anvil so as to ensure contact on the cutting edge on the anvil and thus produce the desired cut. Although the latter mentioned proposed device appears to be more advantageous than prior devices, it has several limitations. They are primarily due to the shape of the knife's cutting edge and the fact that it is only linearly or axially advanced toward the anvil. As a result, the cut is not of optimum shape. Also, sometimes, substantial force is needed to advance the knife to produce the cut. Furthermore, trauma often occurs due to the cutting operation.

## OBJECTS AND SUMMARY

It is an object of the present invention to provide a new and improved abdominal wall aperturing device in which the above-referred to disadvantages are substantially reduced or avoided.

According to the present invention there is provided an abdominal wall aperturing device comprising a body member, an elongated shaft extending out of the body member and axially displacable with respect thereto, a cutting knife having a uniquely shaped cutting edge releasably attached to a free end of the shaft and having a substantially conical portion which tapers axially in the direction of the cutting knife, and means for effecting displacement of the knife toward the anvil in an axial as well as rotational motion.

With such an abdominal wall aperturing device the shaft is introduced into the abdominal cavity through a suitable incision made in the site of the desired aperture and the surgeon, having access to the abdominal cavity, screws the anvil onto the free end of the shaft. The anvil is then displaced towards the abdominal wall and when it has reached the desired position, the knife is caused to move towards the anvil, both axially and rotationally, resulting in the cutting of the aperture. By virtue of the provision of the anvil with the conically tapered portion and other means, the muscles are displaced to either side of the anvil, thereby reducing to a minimum the damage to the muscles during the aperturing. With the device of the present invention minimum trauma to the patient occurs.

Embodiments of annular abdominal wall aperturing devices in accordance with the present invention will now be described by way of example and with reference to the accompanying drawings in which:

## BRIEF DESCRIPTION OF THE DRAWINGS

Figs. 1a and 1b are simplified perspective and planar diagrams, useful in describing a unique knife;

Figs. 2a and 2b are substantially cross-sectional diagrams of a preferred embodiment;

Figs. 3a, 3b and 3c are partial views useful in explaining novel features of the invention;

Figs. 4a and 4b are views of the final aperture, producible with the novel invention;

Figs. 5a, 5b and 5c are partial views useful in explaining a latest developed device with novel features; and

Fig. 6 is a side view of a preferred embodiment of the invention with additional novel features.

## DESCRIPTION OF THE PREFERRED EMBODIMENTS

Before describing the invention some of the disadvantages of the prior devices will be mentioned.

Typically, the thickness of the abdominal wall is on the order of 40 mm. With prior devices it is necessary to compress the wall and reduce its thickness down to approximately 15 mm. To achieve such wall compression, substandial force need be applied. Also, in at least some cases, if not most, such wall compression may cause patient trauma. Even if trauma is not caused by having to compress the wall to the extent necessary, trauma may result from the cutting with a knife which moves only linearly into the wall with its continuous circular cutting edge.

In accordance with the invention an embodiment is provided which, when used, substantially eliminates any likelihood of the occurrence of trauma and does not require large surgeon-provided forces for its use. Briefly, in this embodiment the interposed abdominal wall need be compressed so that it is sufficiently non-flexible. This can be achieved with reasonable surgeon applied rotary forces and without causing trauma. The wall is made sufficiently non-flexible so that a knife which turns about its axis as well as moves linearly toward the anvil produces the cavity with its cutting edge. Preferably the knife's cutting edge is not shaped so that its locus is that of a circle. Rather, it is tooth-like with smooth gradual slopes which produce the actual cut.

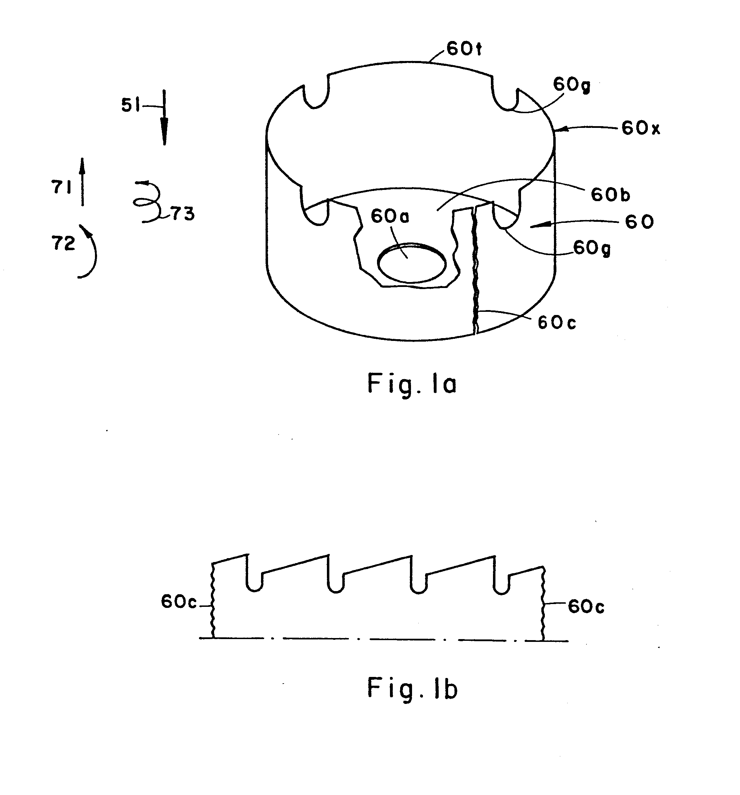

Such a novel knife and its directions of motion will now be explained in connection with Figs. 1a and 1b. The novel knife is designated by numeral 60. It has a base portion 60b and a hole 60a. However, its cutting edge 60x is not one with a circular locus. Rather, the cutting edge 60x is tooth-like, defining a plurality of smooth teeth 60t with grooves 60g between adjacent teeth. If base portion 60b were cut off and the remaining circular knife portion

0141536

cut along line 60c and then laid flat, the cutting edge 60x would appear as shown in Fig. 1b. Such a shape for the cutting edge 60x provides significant advantages as will be explained.

Generically the knife 60 can be thought of as in the shape of a circular cup with a hole in the base center, and with a cup lip which defines the knife's cutting edge which is equidistant from the longitudinal axis of the cup. In case of knife 60 the cutting edge 60x comprises a plurality of teeth of preselected shape, forming the lip of the circular wall of the cup-shaped knife.

However, irrespective of the shape of the cutting edge 60x, the knife 60 is advanced toward an anvil linearly, as represented in Fig. 1a by arrow 71, as well as rotationally as represented by curved arrow 72. The combined motion of the knife 60 is that of a spiral, as represented by 73. The motion and advancement of the knife into the interposed abdominal wall is analogous to that of a screw or bolt being advanced into a body of matter.

It has been discovered that with a knife spirally advanced into the interposed abdominal wall, the latter need be only slightly compressed to be sufficiently non-flexible to enable the screw-like moving knife to cut the cavity therein. Thus, potential trauma is eliminated by not having to fully compress the wall. Since the wall need be only partially compressed the force which the surgeon has to apply is not excessive. Also, since the cut is performed by the spirally advancing knife, the cutting is gradual and smooth, eliminating any likelihood of traumatic effects. A knife such as knife 60 with the toothed cutting edge 60x has been found to be particularly advantageous, to produce an excellent cavity in the wall, smoothly and without likelihood of trauma.

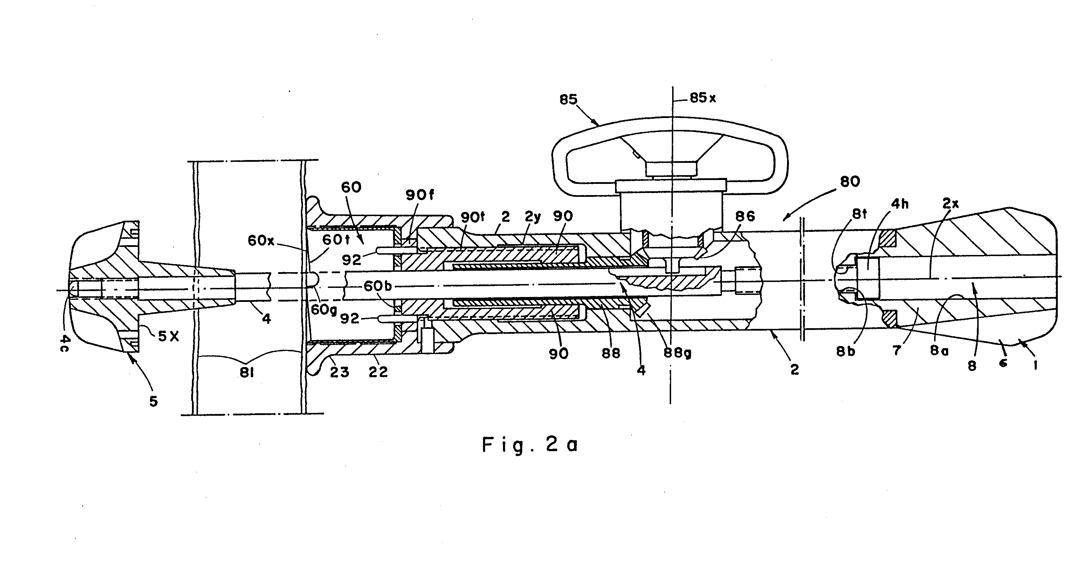

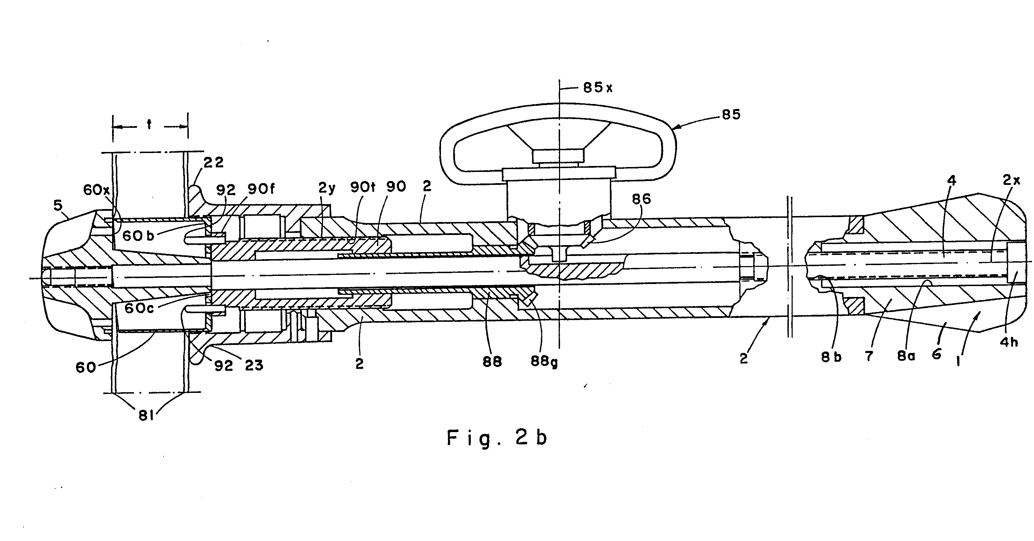

As shown in Figs. 2a and 2b, in accordance with the present invention the abdominal wall aperturing device includes an arrangement for axially displacing an anvil 5 toward a body 2, such as by turning a top handle with wings 6 to axially move shaft 4 into the body, and thus compress the interposed abdominal wall. In addition, the device includes an arrangement, e.g. a second handle 85 which extends out of the body, preferably above the ribbed section and which is coupled by appropriate gears to internal elements, which are in turn attachable to the base portion 60b of novel knife 60. By rotating the second handle, the internal elements, which can be thought of as a hollow bolt, both turn and move axially within the body toward a cartridge 22 in which the knife 60 is located. Thus, as they turn and move axially they cause the knife 60, which is attached to them by pins, to also move out of the cartridge in a rotational as well as linear motion, to cut the cavity in the abdominal wall as herebefore described. Although different mechanical arrangements may be employed to so advance the knife 60 out of its cartridge a specific embodiment will be described, for explanatory purposes and without limiting the invention thereto.

In Fig. 2a the device designated by 80 is shown with an anvil 5 most remote from the device's body 2. The anvil 5 is assumed to be attached to the bayonet end 4c of the shaft 4 inside the patient's body, before any compression of the interposed abdominal wall has started. The thickness of the abdominal wall 81 varies from patient to patient. However, it is typically on the order of 40 mm. Fig. 2b shows the device 80 after the knife 60 has cut through the wall and thus formed the desired circular cavity therein.

When knife 60 is used, the abdominal wall need not be totally compressed. Rather, it need be compressed only down to a thickness t (Fig. 2b) which can be on the order of 15 mm until the wall is sufficiently non-flexible to be cut by knife 60. Thus, with knife 60 the likelihood of trauma occurring is greatly reduced, if not totally eliminated.

0141536

As shown in these Figures the aperturing device 80 includes a body 2 with a handle 1 on top of it at its end, remote from the end at which the knife 60 is located. The handle 1 includes wings, such as wings 6, which radially extend from a tubular member 7 with an axial bore 8, which is counterbored, so that its upper portion 8a is of larger diameter than the lower portion 8b which is threaded as indicated by 8t. The handle 1 with its tubular member 7 are secured to the body 2 so that the handle is rotatable about the body axis but cannot move axially with respect thereto. One can view the tubular member 7 as a nut in body 2 which is rotatable only therein by forces applied by a surgeon to handle 1. Located within the body and the tubular nut-like member is the shaft 4 which is like a bolt. It threadably engages threads 8t and its head 4h is accommodated in member portion 8a. Thus, as the handle is rotated the shaft 4 moves axially either up or down within the body 2.

When used, after the anvil 5 is locked to shaft 4, as herebefore described, the surgeon, to compress the interposed abdominal wall, merely rotates the handle 1, causing the shaft 4 to move up until the wall is sufficiently compressed, e.g. to the thickness t, as shown in Fig. 2b. That the wall has been compressed to the desired thickness t may be indicated to the surgeon by so designing the device so that when the distance between the flat upper surface of the anvil, designated 5x in Fig. 2a and the flange 23 of a knife-enclosing cartridge 22 is equal to t, the head 4h of shaft 4 is flush with the top of handle 1, as shown in Fig. 2b.

Once the interposed abdominal wall 81 is compressed, the cutting of the desired cavity in the wall can start. As previously explained in connection with Fig. 1a in the present embodiment of device 80, the cutting is done with knife 60, which has to be rotated as well as moved axially toward the anvil. Such motion is provided to the knife by rotating a handle 85, which extends out of the housing 2 perpendicular to the housing's longitudinal axis 2x. Handle 85 turns about an axis 85x which is perpendicular to axis 2x. As it turns or rotates it rotates a conical gear 86 inside body 2. Axially located inside the housing is a sleeve 88 with a central bore so that the sleeve can extend axially about shaft 4. The top of the sleeve 88 is shaped to form a conical gear 88g which meshes with gear 86. Thus, as the handle 85 is turned, causing gear 86 to rotate about axis 85x, gear 88g turns, thus rotating sleeve 88 about the body's axis 2x.

As shown in Figs. 2a and 2b, another sleeve 90 is accommodated between the outer surface of sleeve 88 and the inner surface of body 2, which is threaded near its lower end to which cartridge 22 is attached. The threads on the inner surface of body 2 are designated by 2y. The outer surface of sleeve or insert 90 is threaded as indicated by 90t. Threads 90t mesh with those of 2y. Sleeve 90 is coupled to sleeve 88 in a manner whereby when the latter rotates it imparts a rotary motion to sleeve 90. The sleeve 90 in addition to rotating moves axially in the housing, while sleeve 88 can only rotate. As sleeve 90 rotates it also moves axially along axis 2x toward cartridge 22 due to the meshing of teeth 90t and 2y. The end of sleeve 90 closest to the cartridge 22, wherein knife 60 is located, is flanged, as indicated by 90f. Apertures (not designated) aligned with apertures 60h in the base plate 60b of knife 60 are used to enable pins 92 to be inserted into the holes in the flanges end 90f of sleeve 90 and thus releasably attach the knife 60 to sleeve 90.

From the foregoing it should thus be apparent that by rotating handle 85 gear 86 rotates about axis 85x, causing gear 88g and thus sleeve 88 to rotate about the body axis 2x. This rotation causes sleeve 90 to rotate, thus causing the knife 60 to rotate. However, due to the meshing

0141536

of teeth 90t and 2y, as sleeve 90 rotates it also moves axially toward the stationary cartridge 22, thus imparting axial motion to the knife 60 toward anvil 5. The knife 60, as it rotates and moves axially toward the anvil 5, produces a smooth cylindrical cut in the interposed abdominal wall 81. As previously explained in connection with Figs. 1a and 1b, preferably the knife's cutting edge 60x is toothed, as shown by teeth 60t and grooves 60g. Such a shaped cutting edge assures the cutting of a smooth cylindrical cavity in the abdominal wall.

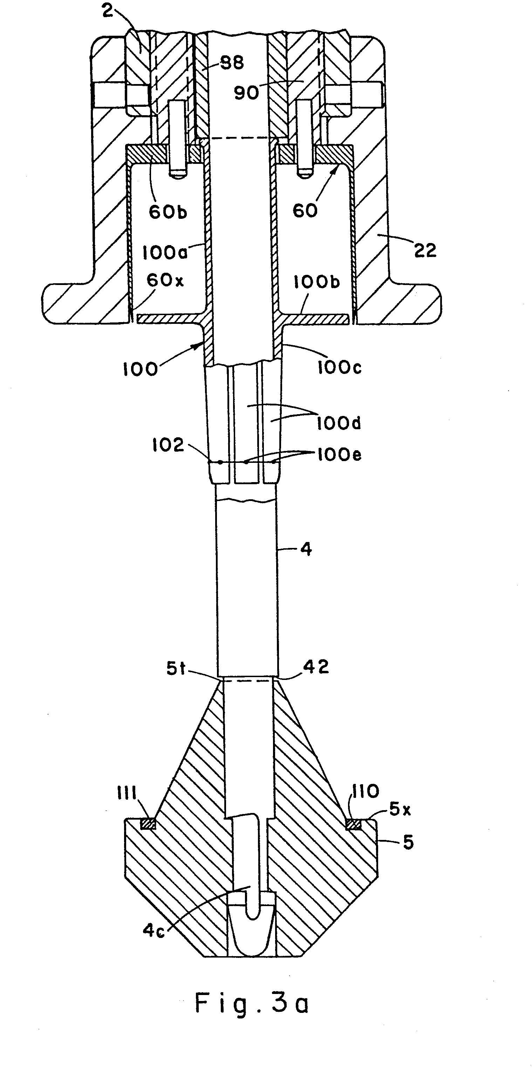

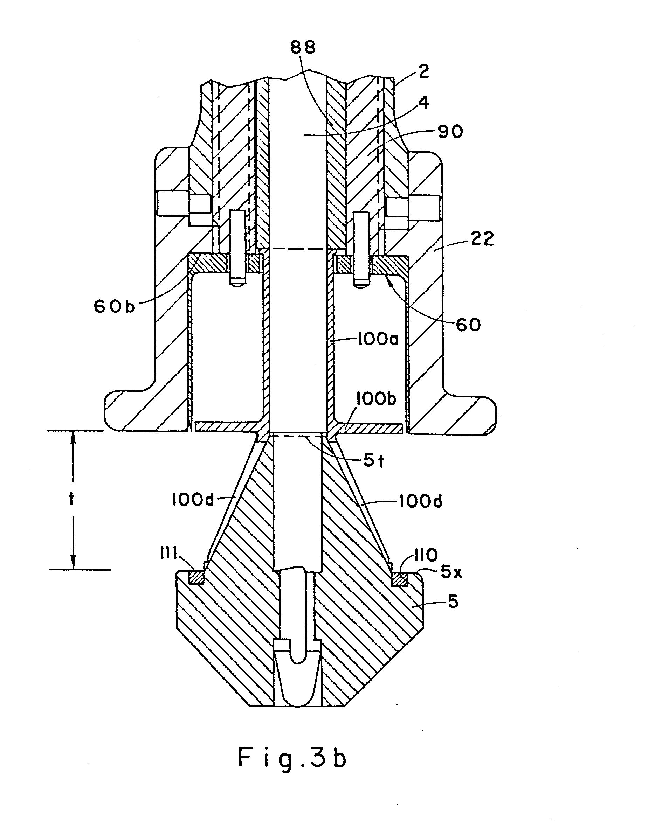

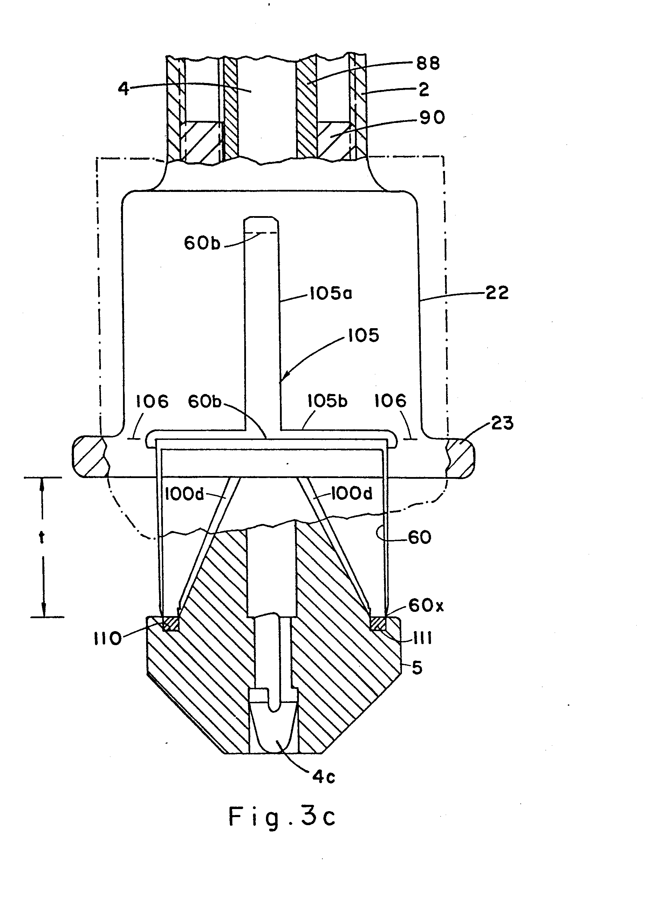

In order to reduce any likelihood of abdominal wall getting squeezed into the cup-shaped knife 60 and getting cut by it, as it is advanced rotatably and axially toward the anvil 5, in a preferred embodiment a novel sleeve and other means are included to prevent such occurrence. This embodiment will be described in connection with Figs. 3a, 3b and 3c. In these Figures, elements like those described will be designated by like numerals. Fig. 3a is similar to Fig. 2a in that it shows the anvil 5 before it is advanced. Fig. 3b shows the anvil at the advanced position, i.e. at the distance t from the main body, but before the knife 60 is lowered, and Fig. 3c is similar to Fig. 2b in that is shows the knife 60 after the cut has been performed.

As shown in these Figures, the bottom end 4c of shaft 4 is shaped to form a bayonet type end to which the anvil 5 can be locked by a partial rotation about the shaft. The shaft is of reduced diameter at 42 and the top of the cone-shaped portion 5t of the anvil 5 has a diameter equal to that of the shaft. Thus, when the anvil is bayonet locked to the shaft its top 5t at the top of the cone has a diameter equal to the larger diameter of the shaft at 42 (see Fig. 3a).

A novel, essentially cylindrical sleeve 100 is included. It may also be referred to as the insert or destructor. It is shown partially in front and cross-sectional views in Fig. 3a. It is essentially cylindrical and is supported around shaft 4. Its upper part 100a

**0141536**

extends from a radial disc or flange 100b of the sleeve through the knife hole 60a around the shaft until it abuts the rotatable element 88. When so located the radial disc 100b occupies a flush position with the cutting edge 60x, thus effectively closing off the knife. However, the diameter of disc 100b is slightly less than the inner diameter of the knife, to enable the latter to extend out of cartridge 22 without obstruction. The disc 100b is flush with the bottom side of the cartridge 22.

The sleeve has a lower portion 100c which extends downwardly from disc 100b. It is also cylindrical except that it has several longitudinal cuts so that it forms a plurality of fingers 100d, circularly arranged around shaft 4. A string 102 is wrapped around the outer ends of the fingers in order to squeeze them against the shaft until they are spread apart as will be explained. In order to prevent the string from becoming disengaged from all the fingers it is threaded and tied to one or more of the fingers through small holes 100e.

As the anvil 5 is advanced toward body 2 and the squeezing of the abdominal wall, matter is prevented from getting squeezed into the knife. When the shaft 4 has been withdrawn sufficiently so that the tope 5t of the anvil 5 reaches the fingers 100d, since its diameter is not greater than that of the shaft 4 the anvil starts advancing between the fingers. Due to its cone shape it starts pushing the fingers apart, causing the string 102 to snap. However, it remains attached to at least one of the fingers. As the anvil is farther advanced, the fingers abut the cone-shaped portion of the anvil, thus pushing abdominal wall matter away from the anvil. Fig. 3b shows the anvil at its most advanced position, in which its top 5t is essentially flush with disc 100b and the bottom of cartridge 22. In this position the fingers 110d extend along the cone-shaped portion of the anvil. The slope of the cone-shaped portion

0141536

of the anvil is chosen so that the tips of the fingers are spread apart by a distance which is less than the knife diameter.

Once the anvil 5 has been raised to the desired position the cutting operation starts by causing the knife to advance axially as well as rotate about its axis to cut any abdominal wall matter between the cartridge and the anvil. The cutting operation has been described herebefore. In order to enable the user to determine the depth of cut being performed, a novel slot is formed in the cartridge, as shown in Fig. 3c. Basically, the slot, designated 105, is in the shape of an inverted T with an axial part 105a and a horizontal part 105b. Before the knife is withdrawn from the cartridge, the knife bottom 60b is viewable at the top of slot part 105a, as represented by the dashed line marked 60b (see Fig. 3c). However, as the cutting operation takes place and the knife is extracted out of the cartridge, its bottom 60b moves down along slot part 105a. At the end of the cut when the knife abuts the anvil as shown in Fig. 3c, the knife bottom 60b appears in the middle of horizontal slot 105b. If desired, markings 106 on the cartridge may be provided to help the user determine when the knife has reached its final travel point. Preferably the bottom side of the disc 100b against which the person's skin is pressed is serrated. Such serrations tend to grab the skin, thus reducing the likelihood that it will tend to twist as the cutting takes place by the turning knife. Also, the bottom side of flange 23 of cartridge may be serrated for the same purpose. Furthermore, the top side 5x of the anvil 5 may be serrated, again for the same purpose, i.e. provide friction to prevent tissue twisting by the knife.

In order not to damage the end of the knife the anvil may include a circular groove 110 in which a ring of a resilient material 111, e.g. nylon, may be inserted to provide a flexible surface for the sharp knife edge to abut against.

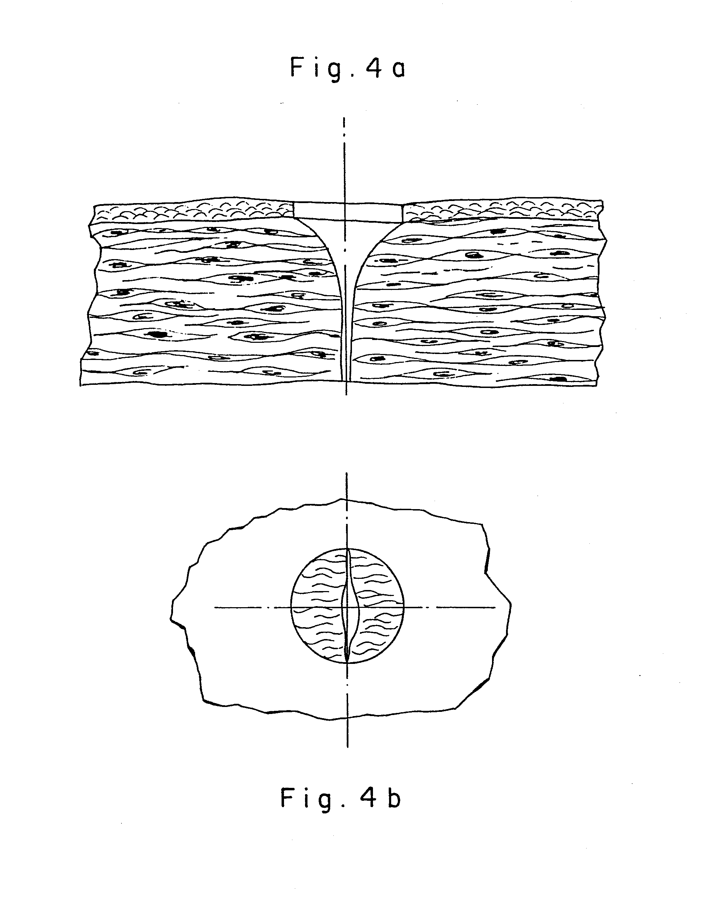

After the cut has been performed, the knife is retracted, and the shaft is extended. Then the surgeon removes the anvil from inside the body through the incision through which it was originally introduced into the body. Then the body 2 is pulled away from the body to remove the shaft therefrom. The resulting cavity is as shown in Figs. 4a and 4b. It should be stressed that in the described embodiments the cartridge 22 and anvil 5 are assumed to be made of molded plastic. They are disposable after each use and replaceable by new ones for subsequent use. The knife 60 on the other hand is typically made of sharpened metal. It may also be disposed of or sterilized for subsequent use. As to the embodiment with novel sleeve 100, since in use its fingers are spread apart, a new sleeve must be used for each operation.

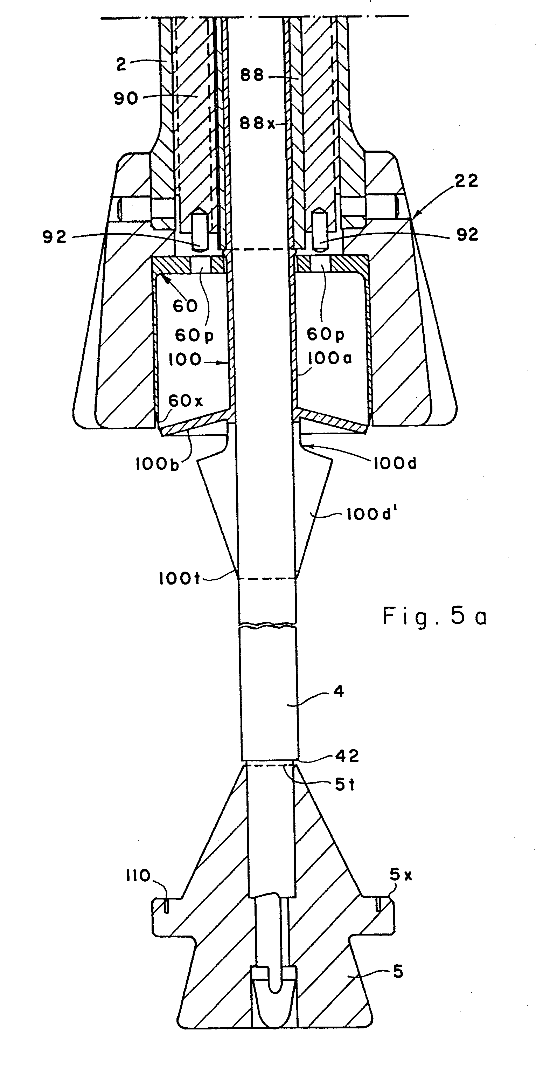

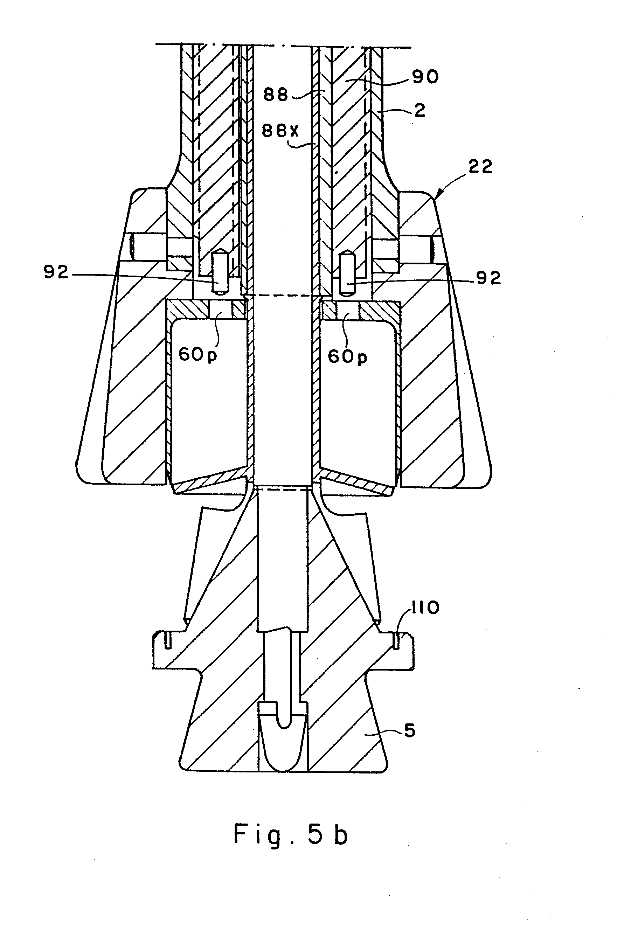

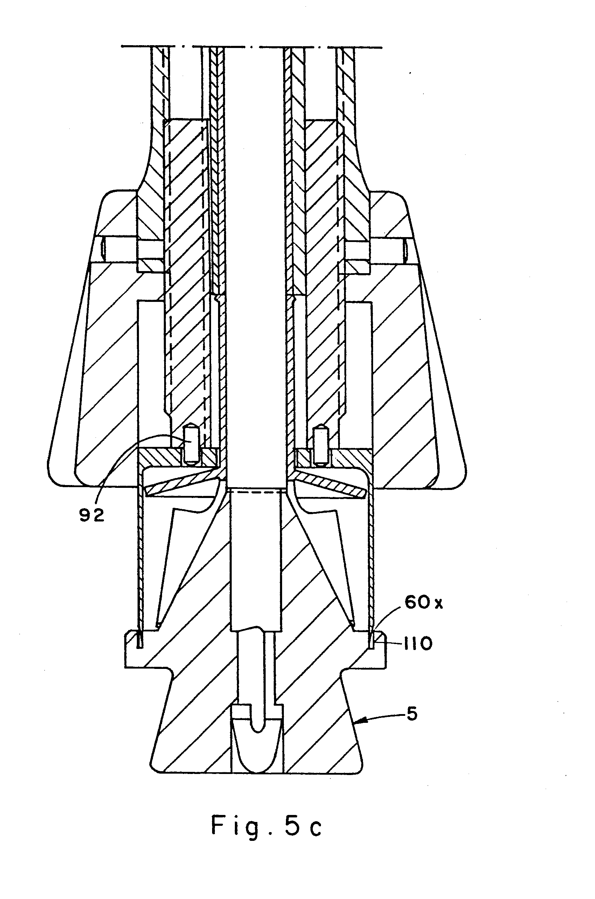

A further embodiment of the present invention will now be described in connection with Figs. 5a, 5b and 5c which are analogous to Figs. 3a, 3b and 3c, respectively, and in which elements previously described are designated by like numerals.

In the prior embodiment the inner surface of sleeve 88 which rotates when handle 85 is turned is juxtaposed the outer surface of shaft 4. Once the anvil has been fully advanced the shaft is under pressure due to the compressed abdominal wall, hereafter simply referred to as the tissue. Only then is sleeve 88 rotated.

If shaft 4 were to come in contact with sleeve 88 one may have to apply large forces to turn the handle 85 in order to rotate sleeve 88. To prevent such a possibility a stationary sleeve 88x is coaxially positioned between shaft 4 and sleeve 88. Sleeve 88x is always stationary with clearance being provided between it and shaft 4 as well as sleeve 88. The top of insert 100 abuts against one end of stationary sleeve 88x. The other end of sleeve 88x abuts against a stationary inner part (not shown) of body 2.

In the presently described embodiment the fingers 100d of insert 100 are not flat. Rather, they are dagger shaped or tooth shaped, as designated by 100d'. Initially their tips 100t are held together by tiny strips of the plastic matter from which the sleeve is molded. However these strips tear as the fingers are forced apart by the cone-shaped portion of anvil 5. Such shaped fingers penetrate into the tissue which is compressed around the cone-shaped portion of the anvil 5, as the latter is pulled toward body 2. Thus, as the cutting takes place by the rotating and axially advancing knife 60 the fingers assist in preventing the tissue from tending to rotate due to the rotating knife. In fact, in order to further prevent the tissue from rotating, all parts which come in contact with it are provided with rough or serrated rather than smooth surfaces. The surfaces are made to be rough to tend to grab the tissue and prevent it from rotating or twisting. These surfaces include the surface 5x of anvil 5, the tissue facing side of disc 100b as well as the bottom side of the flanged cartridge 22.

If desired the resilient material 111 in groove 110 of the anvil 5 may not be included. The knife would then enter the groove 110, as shown in Fig. 5c. As to the disc 100b in the presently described embodiment it is not disc shaped but rather shaped as a truncated cone, thus forming an angle other than 90° with the axis of the insert 100.

In the prior described embodiment the pins 92 which couple the sleeve 90 to the knife 60 extend into the knife through holes, even before the anvil is advanced. See Fig. 3a. Thus, as soon as the sleeve 90 starts to rotate and move axially, like motion is imparted to the knife.

In the presently described embodiment the pins 92 are shorter. Thus, they do not engage and enter the knife through the holes 60p when the anvil 5 is far from the cartridge 22 as shown in Fig. 5a. Even when the anvil is at its most advanced position as shown in Fig. 5b, the pins 92 are not in holes 60p. Thus, during anvil advancement the knife

- 14 -

0141536

is decoupled from sleeve 90. Consequently, there is no danger that any cutting may start by accident. Only after the tissue has been totally compressed and the surgeon atarts turning handle 85 that the pins 92 enter the holes 60p and thus impart the axial and rotational motion to the knife for the cutting operation, as shown in Fig. 5c.

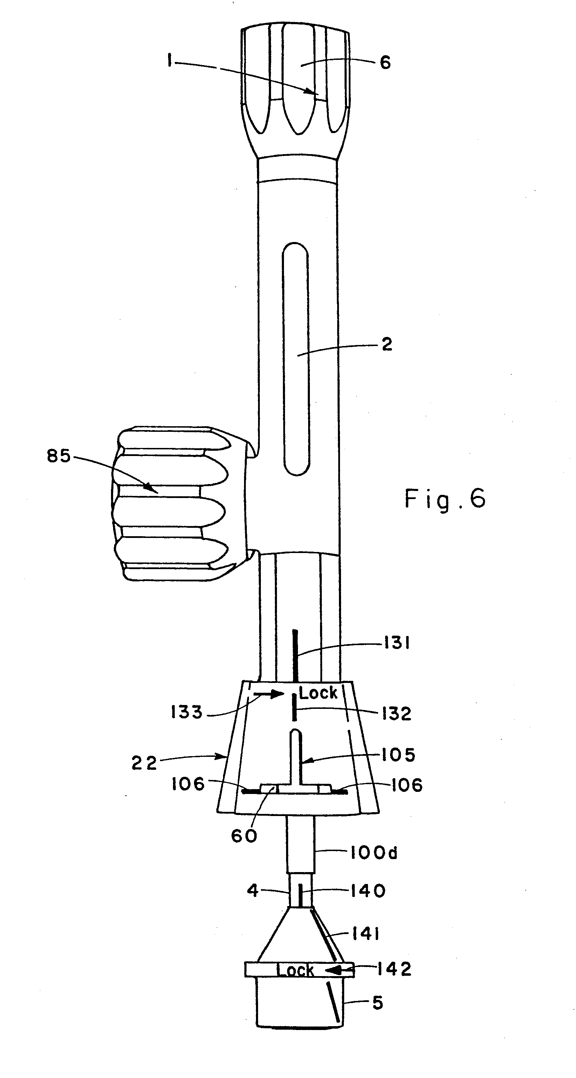

In practice, all parts of the device which may come in contact with the person's tissue may be provided as a sterile disposable package. They may include the anvil 5, the knife 61, the cartridge 22 and the insert 100. Except for the knife the other parts are typically made of molded plastic. A side view of the instrument is shown in Fig. 6.

Some surgeons are not very mechanically adept. In order to assist them special markings are provided. As shown in Fig. 6 an axial marking in the form of a printed line 131 is provided on the body 2 close to the location where the cartridge 22 is to be locked to the body 2. An axial marking 132 is provided on the cartridge as well as an arrow shaped marking 133 and the printed word LOCK. The arrow 133 shows the direction in which the cartridge has to be turned to be locked to the body. Only when the axial markings 131 and 132 are aligned is the cartridge properly locked.

Similarly an axial marking 140 is provided on the shaft 4. It is aligned with marking 131 on body 2. A marking 141 is provided on the cone-shaped part of anvil 5. On it, an arrow shaped marking 142 with the word LOCK is provided. Only when markings 140 and 141 are aligned is the anvil locked onto the shaft 4. As shown the cartridge 22 is properly locked, while the anvil is not. Only when markings 131 and 132 and 140 and 141 are aligned are both parts, i.e. the cartridge and anvil properly connected.

Although particular embodiments of the invention have been described and illustrated herein, it is recognized that modifications and variations may readily occur to those skilled in the art and consequently, it is intended that the claims be interpreted to cover such modifications and equivalents.

CLAIMS:

1.          An abdominal wall aperturing device comprising:

a body member;

an elongated shaft extending out of said body member and axially displaceable with respect thereto;

a cutting knife in the shape of a circular hollow cup with a lip which defines the knife's cutting edge with the knife being releasably held with respect to said body member;

an anvil member releasably attachable to a free end of the shaft and having a substantially conical portion which tapers axially in the direction of said cutting knife; and

actuatable displacing means for effecting relative mutual displacement of said knife and said anvil towards each other so as to ensure contact of said cutting edge on said anvil, said actuatable displacing means comprising a first component displacing means for displacing said anvil member towards said body member and second component displacing means for displacing said knife by axial and rotational motion towards and into contact with said anvil member.

2.          A device according to Claim 1 wherein said first component displacing means comprise a first handle for displacing said anvil member toward said body member by retracting said shaft into said body member to thereby said anvil member toward the end of the body member through which said shaft extends and said second component displacing means includes a second handle for advancing said knife toward said anvil by axial and rotational motion.

3.          A device according to Claim 1 wherein said knife is cup-shaped with a peripheral cutting edge defining a plurality of cutting teeth separated by preselected shaped grooves.

4.          A device according to Claim 3 wherein said knife and said anvil are respectively formed with fluid drainage apertures.

- 16 -

5.        A device according to Claim 1 wherein said knife 0141536 is releasably retained in a cartridge member releasably coupled to an end portion of said body member.

6.        A device according to Claim 5 wherein said cartridge is formed of a cylindrical housing terminating in an outwardly directed flange.

7.        A device according to Claim 6 wherein said cartridge and said body member end portion are formed with respective elements of a bayonet connection.

8.        A device according to Claim 3 wherein the shaft extending out of said body is of a first diameter with a portion near the free end thereof being of a second diameter, smaller than said first diameter, with the anvil member being attachable to the free end of the shaft so that its top abuts the shaft whereat its diameter is reduced, and further including a hollow cylindrical sleeve coaxial with said shaft, said sleeve defining an outwardly extending disc-shaped flange with a first portion of said sleeve in one direction from said flange extending into said body about said shaft so that the flange is substantially flush with the cutting edge of said knife and said sleeve having a second portion extending from said flange away from said knife edge, said second portion defining a plurality of axial fingers extending about the periphery of said shaft of said first diameter, whereby as said anvil advances toward said body and its top reaches the tips of said fingers the conical portion of said anvil spreads the fingers apart.

9.        A device according to Claim 8 wherein said cone portion of said anvil is tapered and the lengths of the fingers chosen so that when said anvil is at a preselected distance from the cutting edge of said knife the distance between the tips of the spread apart fingers is less than the inside diameter of the cutting edge of said knife.

10.        A device according to Claim 9 wherein said knife and said anvil are respectively formed with fluid drainage apertures.

0141536

11.     A device according to Claim 10 wherein said knife is releasably retained in a cartridge member releasably coupled to an end portion of said body member.

12.     A device according to Claim 11 wherein said cartridge is formed of a cylindrical housing terminating in an outwardly directed flange.

13.     A device according to Claim 12 wherein said cartridge and said body member end portion are formed with respective elements of a bayonet connection.

14.     A device according to Claim 9 wherein said knife is releasably retained in a cartridge with said cartridge and said body member end portion being formed with respective elements of a bayonet connection, and wherein said anvil and the free end of said shaft are formed with respective elements of a bayonet lock, and wherein said body mamber, said cartridge, said shaft and anvil are provided with markings to provide an indication when said cartridge is properly bayonet connected to said body and said anvil is properly bayonet connected to said shaft.

15.     A device according to Claim 3 wherein the shaft extending out of said body is of a first diameter with a portion near the free end thereof being of a second diameter, smaller than said first diameter, with the anvil member being attachable to the free end of the shaft so that its top abuts the shaft whereat its dimaeter is reduced, and further including a hollow cylindrical sleeve coaxial with said shaft, said sleeve defining an outwardly extending disc-shaped flange with a first portion of said sleeve in one direction from said flange extending into said body about said shaft so that the flange is substantially flush with the cutting edge of said knife and said sleeve having a second portion extending from said flange away from said knife edge, said second portion defining a plurality of axial fingers extending about the periphery of said shaft of said first diameter, whereby as said anvil advances toward said body and its top reaches the tips of said fingers the conical portion of said anvil spreads the fingers apart, with said disc shaped flange being at an angle other than 90° with respect to the cylindrical sleeve.

16. A device according to Claim 6 wherein the bottom side of the flange of said cartridge and the top surface of said anvil are substantially serrated to reduce abdominal wall twisting as said knife cuts through said wall.

Fig. 1a

Fig. 1b

0141536

Fig. 2 a

Fig. 2 b

Fig. 3a

Fig.3b

Fig.3c

Fig. 4a

Fig. 4 b

Fig. 5 a

Fig. 5b

Fig. 5c

Fig.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 776 237 (HILL et al.)<br>* column 2, lines 25-50; figures * | 1,3 | A 61 B 17/32 |
| A | US-A-4 074 432 (ALFIERI)<br>* column 2, lines 25-33; figure 3 * | 1 | |
| A | US-A-3 701 352 (BOSWORTH)<br>* figure 1 * | 1 | |
| A | US-A-3 902 498 (NIEDERER)<br>* column 5, lines 37-44; figures * | 1 | |
| A | US-A-1 540 860 (POBANZ)<br>* page 1, lines 52-67; figure 2, ref. 19 * | 1 | **TECHNICAL FIELDS SEARCHED (Int. Cl.4)**<br><br>A 61 B<br>B 26 F<br>B 21 D |
| A | DE-A-1 006 117 (SCHREIBER) | | |
| A | DE-A-2 037 504 (TJONG-JOE-WAI) | | |

The present search report has been drawn up for all claims

| Place of search | Date of completion of the search | Examiner |
|---|---|---|
| THE HAGUE | 21-01-1985 | GLAS J. |